# FASCICULE DE BREVET EUROPEEN

(11) **EP 0 742 833 B1**
(45) Date de publication et mention de la délivrance du brevet: **31.03.2004**
(21) Numéro de dépôt: 95907723.1
(22) Date de dépôt: 31.01.1995
(51) Int. Cl.: C12N 15/86, C07K 16/28, C12N 7/01, C12N 5/10, C12N 15/13

(54) **BACULOVIRUS RECOMBINANT ET SON UTILISATION POUR LA PRODUCTION D'ANTICORPS MONOCLONAUX**
REKOMBINANTER BAKULOVIRUS UND SEIN VERWENDUNG ZUR HERSTELLUNG MONOKLONALER ANTIKÖRPER
RECOMBINANT BACULOVIRUS AND USE THEREOF IN THE PRODUCTION OF MONOCLONAL ANTIBODIES

(30) Priorité: 31.01.1994 FR 9401015
(43) Date de publication de la demande: 20.11.1996
(73) Titulaire: Institut National de la Recherche Agronomique (INRA), 75338 Paris Cédex 07 (FR); CENTRE NATIONAL DE LA RECHERCHE SCIENTIFIQUE, 75016 Paris Cédex (FR)
(72) Inventeur: CERUTTI, Martine, F-30380 Saint-Christol-lès-Alès (FR); CHAABIHI, Hassan, F-30100 Alès (FR); DEVAUCHELLE, Gérard, F-30380 Saint-Christol-lès-Alès (FR); GAUTHIER, Laurent, F-30100 Alès (FR); KACZOREK, Michel, F-34980 Montferrier (FR); LEFRANC, Marie-Paule, F-34830 Clapiers (FR); POUL, Marie-Alix, F-34000 Montpellier (FR)
(74) Mandataire: Pernez, Helga
(86) Numéro de dépôt international: PCT/FR1995/000110
(87) Numéro de publication internationale: WO 1995/020672

(56) Documents cités:
- WO-A-90/14428
- WO-A-92/05264
- WO-A-92/05265
- WO-A-93/09238
- BIOTECHNOLOGY, vol.10, Août 1992, NEW YORK US pages 910 - 912 REIS, U. ET AL. 'Antibody production in silkworm cells and silkworm larvae infected with a dual recombinant Bombyx mori nuclear polyhedrosis virus' cité dans la demande
- BIOTECHNOLOGY, vol.8, Juillet 1990, NEW YORK US pages 651 - 654 ZU PUTLIZ, J. ET AL. 'Antibody production in baculovirus infected insect cells' cité dans la demande
- PROCEEDINGS OF THE NATIONAL ACADEMY OF SCIENCES OF USA., vol.87, Mai 1990, WASHINGTON US pages 3942 - 3946 HASEMANN, C. A. & CAPRA, J.D. 'High-level production of a functional immunoglobulin heterodimer in a baculovirus expression system' cité dans la demande
- POUL ET AL IMMUNOTECHNOLOGY vol. 1, 1995, pages 189 - 196

## Description

La présente Invention est relative à un baculovirus modifié et à son utilisation pour la production d'immunoglobulines.

Les anticorps ou immunoglobulines sont produits par les lymphocytes B. Chaque lymphocyte B sécréte un seul type d'anticorps. Chaque molécule d'immunoglobuline est constituée de l'association de deux chaînes lourdes (H) et deux chaînes légères (L) reliées par des ponts disulfures. Chaque chaîne est constituée d'une partie variable (VH et VL) qui possède le site de fixation à l'antigène et d'une partie constante (CH et CL). Il existe plusieurs types de chaînes lourdes (γ1, γ2, γ3, γ4, α, ε, µ) qui définissent les différentes classes d'immunoglobulines (IgG1, IgG2, IgG3, IgG4, IgA, IgE, IgM...) et deux types de chaînes légères : (chaîne kappa (κ) et chaîne lambda (λ). Par exemple les anticorps de classe IgG1 sont constitués de deux chaînes lourdes de type γ1 et de deux chaînes légères κ ou lambda λ. Les parties variables sont le support de la spécificité de l'anticorps pour son antigène.

Pour chaque chaîne d'un anticorps donné, la région variable est constituée de plusieurs domaines dont certains sont plus ou moins conservés. Le réarrangement de ces régions variables est le fruit d'une recombinaison au niveau de l'ADN génomique des lymphocytes B.

Les anticorps monoclonaux sont classiquement produits à partir de cultures de lignées d'hybridomes, chaque lignée, dérivée d'un seul lymphocyte B, sécrétant un seul type d'immunoglobuline.

Les anticorps monoclonaux (MAbs) sont utilisés couramment aujourd'hui pour le diagnostic in vitro, et leur utilisation en thérapie et pour le diagnostic in vivo connait un développement prometteur. Ce développement est toutefois freiné par le fait que les seuls anticorps monoclonaux dont on puisse disposer relativement facilement et en quantité suffisante à partir des cultures d'hybridomes sont des anticorps monoclonaux de rongeurs. Or, les immunoglobulines de rongeurs (et de manière générale les immunoglobulines non-humaines) induisent chez l'homme une réponse immune indésirable, ce qui limite considérablement leur intérêt thérapeutique.

De nombreuses recherches ont été effectuées dans le but d'obtenir des immunoglobulines ne possédant pas cet inconvénient ; il a en particulier été proposé de fabriquer par génie génétique des anticorps recombinants, dans lesquelles la plus grande partie possible de la molécule est dérivée d'un gène d'origine humaine.

Les anticorps obtenus, dans lesquels seuls les domaines variables sont d'origine non-humaine, sont dénommés anticorps chimériques. Il existe également des anticorps dits humanisés, dont les séquences des régions variables non directement impliquées dans la reconnaissance de l'antigène ont été remplacées par des séquences d'origine humaine. Dans les deux cas, la majeure partie de la molécule d'immunoglobuline est dérivée d'un gène d'origine humaine.

L'obtention d'anticorps par génie génétique nécessite toutefois le choix d'un hôte d'expression approprié, assurant les modifications post-traductionnelles nécessaires pour reproduire les propriétés de l'anticorps natif. Dans ce but il a été proposé, entre autres, d'utiliser le système baculovirus/cellules d'insectes.

Les baculovirus sont utilisés courammment comme vecteurs pour l'expression de gènes hétérologues, placés sous contrôle des promoteurs viraux, dans des cellules d'insectes infectées. Le promoteur de la polyédrine ou bien celui de la p10 (protéines produites en grande quantité pendant la phase très tardive du cycle de réplication viral) sont ainsi fréquemment utilisés dans ce but.

HASEMAN et CAPRA [Proc. Natl. Acad. Sci. USA, 87, 3942-3946 (1990)], PUTLITZ et al. [Bio/Technology, 8, 651-654, (1990)], REIS et al. [Bio/Technology, 10, 910-912, (1992)] ont ainsi construit des baculovirus dans lesquels étaient insérées en un même locus, deux copies du promoteur de la polyédrine, l'une de ces copies contrôlant l'expression d'un gène codant pour la chaîne lourde d'une immunoglobuline de souris, l'autre contrôlant l'expression d'un gène codant pour la chaîne légère de la même immunoglobuline. Les cellules d'insectes infectées par ces baculovirus ont sécrété des immunoglobulines possédant sensiblement les mêmes propriétés que les anticorps d'origine lymphocytaire, qui ont servi de modèle. Cependant, la structure des baculovirus modifiés de la sorte ne se maintient pas au-delà de quelques cycles de réplication virale.

La présente Invention a pour but de produire à la fois la chaîne lourde (H) et la chaîne légère (L) d'un anticorps donné dans une cellule d'insecte, en utilisant un vecteur d'expression dérivé d'un baculovirus, ne présentant pas les inconvénients des vecteurs d'expression du même type utilisés dans l'art antérieur pour la production d'immunoglobulines.

Dans ce but les Inventeurs ont construit des baculovirus double-recombinant dans lesquels les séquences codantes des deux chaînes H et L sont situées à des locus différents du génome dudit baculovirus, et chacune est placée sous le contrôle d'un promoteur fort différent, (contrairement aux baculovirus de l'art antérieur cité ci-dessus, où les deux chaînes sont placées dans un même locus du génome d'un baculovirus, et sous contrôle de deux copies du même promoteur).

La présente Invention a pour objet un baculovirus recombinant, caractérisé en ce qu'il comprend :
- une cassette d'expression comprenant une séquence codant pour au moins une partie d'une chaîne H d'une immunoglobuline, laquelle séquence est placée sous contrôle transcriptionnel d'un premier promoteur de baculovirus, et ;
- une cassette d'expression comprenant une séquence codant pour au moins une partie d'une chaîne L d'une immunoglobuline, laquelle séquence est placée sous contrôle transcriptionnel d'un second promoteur de baculovirus ;
le premier et le second promoteur étant deux promoteurs différents, et étant situés dans deux locus différents.

Selon un mode de réalisation préféré de la présente Invention, le premier et le second promoteur sont des promoteurs forts.

Selon un autre mode de réalisation préféré de la présente Invention, l'un des promoteurs est situé à l'emplacement occupé chez le baculovirus sauvage, par le promoteur de la polyédrine et l'autre est situé à l'emplacement occupé chez le baculovirus sauvage, par le promoteur de la P10.

On entend par : "promoteur de baculovirus" tout promoteur qui peut être intégré dans le génome d'un baculovirus, et fonctionner en cellules d'insectes ; un tel promoteur est dit "fort" lorsqu'il permet d'obtenir un niveau de transcription élevé (par exemple, de l'ordre de celui obtenu avec les promoteurs de la polyédrine ou de P10) d'un gène placé sous son contrôle.

Cette définition englobe non seulement les promoteurs de type "sauvage" tels que les promoteurs de la polyédrine et de P10 des baculovirus AcMNPV ou S1MNPV, mais également les dérivés de promoteurs, résultant de modifications plus ou moins importantes de la séquence d'un promoteur "sauvage" de baculovirus, et en particulier les promoteurs synthétiques ou recombinants, tels que par exemple le promoteur synthétique décrit par WANG et al, [Gene, 100, 131-137, (1991)].

Selon un mode de réalisation préféré d'un baculovirus recombinant conforme à la présente Invention, au moins l'un des promoteurs mis en oeuvre est choisi dans le groupe constitué par :
- le promoteur de la polyédrine ;
- le promoteur de la p10 ;
- un nouveau promoteur synthétique, dénommé ci-après promoteur Syn, et constitué par un fragment d'ADN double brin dont la séquence est la suivante :

La séquence du brin (+) de ce fragment est identifiée dans la liste des séquences en annexe sous le numéro SEQ ID NO : 1

La séquence du brin (-) de ce fragment est identifiée dans la liste des séquences en annexe sous le numéro SEQ ID NO : 2

L'utilisation du promoteur Syn permet d'augmenter de manière très significative la production d'anticorps recombinants.

Le promoteur Syn, en tant que tel, fait également partie de l'objet de la présente invention.

Les promoteurs P10, polyédrine, et Syn peuvent être associés 2 à 2 selon n'importe quelle combinaison ; en particulier, le promoteur Syn peut être associé avec avec le promoteur de la polyédrine, ou bien, avantageusement, avec celui de la protéine P10.

Selon un autre mode de réalisation préféré de la présente Invention, chaque cassette d'expression comprend : (i) un promoteur fort de baculovirus, tel que défini ci-dessus ; (ii) une séquence codant pour un peptide-signal ; (iii) une séquence codant pour un domaine variable d'une chaîne H ou L d'immunoglobuline : (iv) une séquence codant pour au moins une partie d'un domaine constant d'une chaîne H ou L d'immunoglobuline.

Un tel baculovirus recombinant constitue un vecteur d'expression directement utilisable pour la production d'immunoglobulines dans une cellule d'insecte.

De préférence, la séquence codant pour le peptide-signal qui est placée sous contrôle du premier promoteur et la séquence codant pour le peptide-signal qui est placée sous contrôle du second promoteur sont deux séquences différentes.

Des séquences connues en elles-mêmes, codant pour des peptides signal fonctionnels en cellules d'insecte sont utilisables pour la mise en oeuvre de la présente Invention. A titre d'exemples non limitatifs, on citera des séquences codant pour les peptides signal de l'acétylcholinestérase de Drosophile, de la trophoblastine ovine, de la lactotransferrine bovine, des chaines H et L d'immuno-globulines, etc...

Les Inventeurs ont toutefois constaté qu'il était préférable, pour obtenir la meilleure sécrétion de la molécule d'immunoglobuline, que la séquence His-Val-Ser soit présente immédiatement en amont du site de coupure utilisé par la signal-peptidase.

Les séquences codant pour les domaines constants et variables peuvent être de même origine, ou d'origine différente ; il peut s'agir également de séquences synthétiques ou recombinantes. Avantageusement, la séquence codant pour le domaine constant est d'origine humaine ; la séquence codant pour le domaine variable peut être d'origine totalement humaine ou au moins partiellement non-humaine, par exemple d'origine murine, etc ....

La présente Invention englobe les cellules d'insecte infectées avec un baculovirus recombinant conforme à l'invention.

L'infection des cellules par un baculovirus double-recombinant conforme à l'Invention résulte dans la production des chaînes H et L. Ces chaînes s'assemblent pour reconstituer l'anticorps monoclonal désiré qui est par la suite sécrété dans le milieu de culture.

La présente Invention a également pour objet un procédé de préparation d'une immunoglobuline, caractérisé en ce que l'on met en culture des cellules d'insecte infectées avec un baculovirus recombinant conforme à l'invention, et en ce que l'on extrait ladite immunoglobuline du milieu de culture.

La présente Invention englobe également les immunoglobulines susceptibles d'être obtenues par le procédé ci-dessus.

La présente Invention a en outre pour objet un procédé de préparation d'un baculovirus recombinant, lequel procédé est caractérisé en ce que :
- l'on prépare un premier plasmide de transfert comprenant une séquence codant pour au moins une partie de chaîne H d'immunoglobuline, sous contrôle transcriptionnel d'un premier promoteur fort d'un baculovirus ;
- l'on prépare un second plasmide de transfert comprenant une séquence codant pour au moins une partie de chaîne L d'immunoglobuline, sous contrôle transcriptionnel d'un second promoteur fort de baculovirus ;
le premier et le second promoteur étant deux promoteurs différents ;
et l'on procède à la recombinaison homologue de l'un et de l'autre desdits plasmides avec l'ADN d'un baculovirus.

La construction d'un baculovirus recombinant conforme à l'Invention se fait en utilisant les techniques classiques de clonage de gènes hétérologues chez les baculovirus.

Schématiquement, la construction des plasmides de transfert se fait en insérant dans un plasmide capable de se répliquer chez un hôte bactérien (en général *E*. *coli*), la région du gène de baculovirus (par exemple p10 ou polyédrine) à la place duquel on souhaite insérer les gènes codant pour les chaînes H ou L d'immunoglobuline. Dans cette région, la séquence codante du gène de baculovirus (et éventuellement la séquence promoteur dudit gène) est remplacée par la séquence codant pour la chaîne d'immunoglobuline à exprimer (et éventuellement par la séquence du promoteur sous contrôle duquel on souhaite exprimer cette chaîne d'immunoglobuline, s'il s'agit par exemple d'un promoteur "dérivé"). Le plasmide de transfert ainsi obtenu contient donc un insert comprenant une séquence hétérologue flanquée de séquences de baculovirus. On cotransfecte ensuite les cellules d'insecte avec l'ADN du vecteur de transfert ainsi réalisé et l'ADN du baculovirus, ce qui par recombinaison homologue entre l'ADN viral et les séquences de baculovirus flanquant la séquence hétérologue dans le plasmide, permet le transfert de la séquence étrangère du plasmide vers le génome viral.

Selon un mode de mise en oeuvre préféré du procédé conforme à la présente Invention, les plasmides de transfert utilisés portent un insert comprenant une cassette d'expression telle que définie ci-dessus, et de part et d'autre de cette cassette, des séquences de baculovirus homologues de celles des régions flanquant la portion du génome viral en remplacement de laquelle on souhaite insérer ladite cassette.

Selon une disposition préférée de ce mode de mise en oeuvre, lesdites séquences de baculovirus sont homologues de celles des régions flanquant le gène de la p10, ou homologues de celles des régions flanquant le gène de la polyédrine.

Après réplication de l'ADN viral dans les cellules transfectées, l'on procède à la sélection des baculovirus recombinants ayant intégré les séquences hétérologues.

Selon un mode de réalisation particulièrement avantageux du procédé conforme à l'invention, l'ADN de baculovirus avec lequel est effectuée la recombinaison homologue des plasmides de transfert est constitué par l'ADN d'un baculovirus préalablement modifié par insertion de deux sites Bsu36I de part et d'autre de la séquence codant pour la protéine P10 (ces deux sites étant les seuls pour l'enzyme concernée dans le génome dudit baculovirus modifié), et digéré par l'enzyme Bsu36I.

Le baculovirus dans lequel sont insérés les sites Bsu36I peut être un baculovirus sauvage, ou un baculovirus déjà modifié, par exemple le virus dénommé AcD3, modifié par délétion du gène et du promoteur polyédrine, à condition toutefois que ce baculovirus conserve les régions flanquantes de la polyédrine et de la P10.

Lors de la mise en présence des fragments d'ADN résultant de la digestion Bsu36I avec les deux vecteurs de transfert (par co-transfection), seuls parmi ces fragments d'ADN, ceux qui recombinent avec le vecteur portant les régions flanquantes de la P10 reconstituent un génome viral circulaire, et permettent d'obtenir des virus viables.

Parmi les virus obtenus, seuls ceux qui ont également recombiné avec le vecteur portant les régions flanquantes de la polyédrine permettent l'expression des deux chaînes d'immunoglobuline, ils peuvent donc être sélectionnés en détectant l'immunoglobuline produite (par exemple par ELISA). En outre, dans le cas où le virus de départ comprend le gène de la polyédrine, seuls les virus qui ont recombiné avec le vecteur portant les régions flanquantes de la polyédrine sont dépourvus de ce gène et peuvent être sélectionnés sur la base de leur phénotype ob- (absence de polyèdres).

Ce procédé permet donc d'obtenir, en une seule étape, en procédant à une triple transfection, les vecteurs ayant intégré les chaînes lourdes et les chaînes légères.

La présente Invention sera mieux comprise à l'aide du complément de description qui va suivre, qui se réfère à des exemples de préparation de baculovirus recombinants conformes à l'invention, et à leur utilisation pour la production d'immunoglobulines dans des cellules d'insectes.

Il doit être bien entendu toutefois que ces exemples sont donnés uniquement à titre d'illustration de l'objet de l'Invention dont ils ne constituent en aucune manière une limitation.

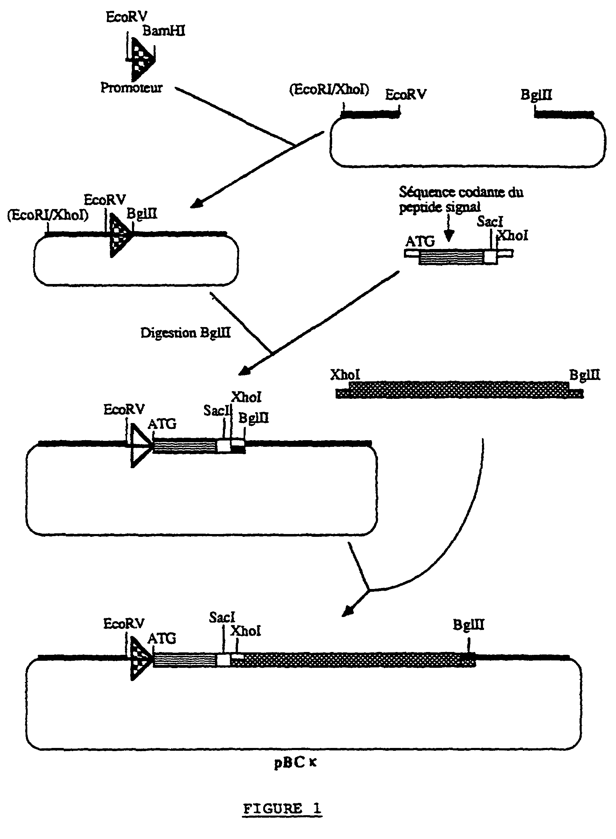

### Exemple 1. : Construction d'une cassette chaîne légère Kappa, (pBCκ) (Figure 1) :

### a- Plasmide pGmAc116T :

Ce vecteur de transfert est dérivé du plasmide pGmAc115T [ROYER et al., J. Virol., 66, 3230-3235, (1992)], lui-même dérivé du plasmide pAcl [CHAABIHI et al., J. Virol., 67, 2664-2671 (1993)] contenant le fragment EcoRI-I du baculovirus de la polyédrose nucléaire d'*Autographa californica* (AcMNPV), et donc le gène polyédrine, et les séquences flanquant ledit gène. Pour obtenir pGmAc116T, le plasmide pGmAc115T a été délété d'un fragment de 1900pb allant d'un site EcoRI situé en amont du gène polyédrine à un site XhoI situé 1900pb en aval de ce site EcoRI. La délétion a été effectuée par coupure exhaustive XhoI, suivie d'une coupure partielle par EcoRI. 5 µg du plasmide pGm115T ont été digérés pendant 2 heures à 37°C par 15 unités d'enzyme XhoI (Boehringer), dans un volume réactionnel de 50 µl et dans les conditions préconisées par le fournisseur. L'enzyme a été éliminée par une extraction au phénol/chloroforme et l'ADN plasmidique a été précipité à l'alcool. Cet ADN a été ensuite partiellement coupé par EcoRI (Boehringer) dans un volume réactionnel de 50 µl en présence de 0,5 unité d'enzyme. L'incubation a été faite à 37°C pendant 20 minutes. Après une nouvelle extraction au phénol/chloroforme, les extrémités générées par les coupures XhoI et EcoRI ont été rendues franches par l'enzyme de Klenow (Biolabs) en présence des 4 dNTPs selon le protocole préconisé par le fournisseur. L'ADN plasmidique a enfin été précipité à l'alcool et incubé avec la ligase du phage T4 (Boehringer) dans les conditions préconisées. Des bactéries *E. coli* compétentes ont été transformées par une partie du mélange de ligation, le criblage des colonies issues de cette transformation a permis de sélectionner le plasmide pGmAc116T.

### b- Les promoteurs :

Le promoteur p10 ou le promoteur polyédrine du virus de la polyédrose nucléaire de *Spodoptera littoralis* (SlMNPV) sont amplifiés par PCR en utilisant des amorces permettant de reconstituer un site EcoRV en amont du promoteur, et un site BglII en aval. Le produit d'amplification est digéré par EcoRV et BglII, et les fragments portant les séquences promotrices sont insérés dans pGmAc116T préalablement digéré par ces mêmes enzymes. La digestion par EcoRV et BglII permet d'éliminer le promoteur polyédrine de AcMNPV, et de le remplacer par l'un des deux promoteurs cités plus haut.

Les plasmides obtenus sont respectivement appelés pGmAc10 (promoteur de la p10), ou pGmAc33 (promoteur de la polyédrine).
Le promoteur synthétique a été produit par synthèse chimique sous forme des deux oligonucléotides complémentaires SEQ ID NO : 1 et SEQ ID NO : 2.
Une fois appariés, ces oligonucléotides reconstituent un ADN double brin directement utilisable dans une réaction de ligation.
Le plasmide pGmAc116T a été digéré par EcoRV et BglII pour éliminer le promoteur polyédrine de AcMNPV, et la séquence du promoteur synthétique a été insérée en remplacement. Le plasmide obtenu est appelé pGmAcSyn.

### c- Peptide-signal :

La séquence codante choisie pour le peptide-signal est la suivante :

Cette séquence a été choisie à partir des séquences publiées par [NEUBERGER M.S., EMBO J, vol. 2, p. 1373-1378 (1983)].

Elle est identifiée dans la liste des séquences en annexe sous le numéro SEQ ID NO : 3

Cette séquence a été synthétisée chimiquement sous forme de deux oligonucléotides complémentaires ayant des extrémités permettant l'insertion du duplex dans un site BglII. A l'une des extrémités du duplex, il y a une séquence correspondant à celle du début de la "charpente 1" (framework 1) des chaînes légères (avec le site SacI), suivie d'une séquence portant un site XhoI. Pour l'appariement, 15 µg de chacun des deux oligonucléotides sont incubés dans 50 µl de tampon (Tris 1 mM pH 7,5, EDTA 0,1 mM), pendant 5 minutes dans un bain marie à 70°C. Le bain est ensuite laissé refroidir jusqu'à température ambiante (22 à 25°C). Le mélange est utilisé directement dans les réactions de ligation avec les plasmides pGmAc10, pGmAc33, ou le plasmide pGmAcSyn préalablement coupés par BglII.

Les conditions de ligation sont les suivantes :

1µg du plasmide pGmAc choisi, coupé par BglII, 1µg de l'oligonucléotide bicaténaire portant la séquence codant pour le peptide-signal, 2µl de tampon ligase 10X (BOEHRINGER), eau distillée q.s.p. 19µl, 1 unité (1µl) de ligase (BOEHRINGER) ; l'incubation est effectuée à 22°C pendant 2 heures ; le produit de ligation est utilisé pour transformer des bactéries *E.coli* compétentes.

### d- Région constante :

La séquence codante de la région constante de la chaîne légère κ humaine a été amplifiée par PCR en utilisant comme matrice de l'ADNc de lymphocytes B humains. Les lymphocytes humains (environ 5x10⁸) ont été préparés à partir de 200 ml de sang en utilisant HISTOPAQUE® (SIGMA). L'ARN total a été extrait de ces lymphocytes en utilisant un kit PHARMACIA (RNA extraction kit). Le premier brin d'ADNc a été préparé à partir de l'ARN total à l'aide du kit "First-Strand cDNA synthesis kit" de PHARMACIA.

Les amorces utilisées pour amplifier le cDNA Cκ sont les suivantes :
* HuCκBAC : (le site XhoI est souligné).
   Cette amorce correspond à une séquence consensus en 3' des séquences codant pour les domaines variables des chaînes légères d'immunoglobulines humaines (Jκ), et contient un site de coupure par XhoI.
   Elle est identifiée dans la liste des séquences en annexe sous le numéro SEQ ID NO : 4
* HuCκFOR : (le site BglII est souligné).

Cette amorce est complémentaire de l'extrémité 3' des gènes Cκ humains et apporte un site BglII en aval du codon stop TAG.

Elle est identifiée dans la liste des séquences en annexe sous le numéro SEQ ID NO : 5

L'amplification avec les amorces HuCκBAC et HuCκFOR a permis d'obtenir un fragment d'environ 340 pb contenant la totalité de la région Cκ encadrée des sites XhoI et BglII.

Le produit de l'amplification a été digéré par BglII et XhoI avant d'être cloné dans les sites XhoI-BglII des plasmides pGmAc portant la séquence codant pour le peptide-signal, pour donner les plasmides pBCκ.

La composition du mélange. de ligation est la suivante :
1µg du plasmide pGmAc coupé par XhoI et BglII ; 200 ng du fragment Ck amplifié et digéré par BglII et XhoI, 2µl de tampon ligase 10X (BOEHRINGER), eau distillée q.s.p. 19µl, 1 unité (1µl) de ligase (BOEHRINGER).

L'incubation est effectuée à 22°C pendant 2 heures ; le produit de ligation est utilisé pour transformer des bactéries *E.coli* compétentes.

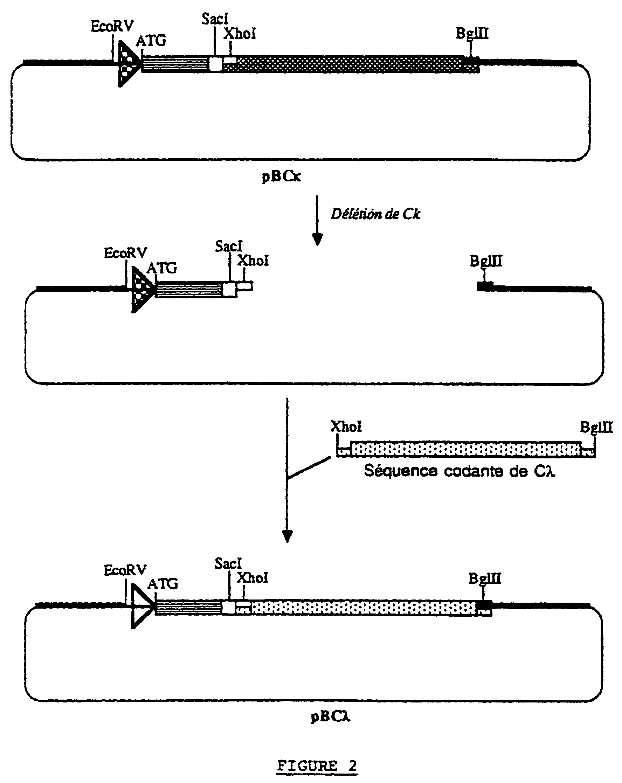

### Exemple 2 - Cassette chaîne légère Lambda (pBCλ) (figure 2) :

### a. Région constante Cλ :

Comme pour la séquence codante de la région constante Ck, la séquence codante Cλ a été obtenue par amplification par PCR de l'ADN complémentaire des ARN messagers de lymphocytes B humains.

L'amplification par PCR de la région Cλ a été réalisée en présence de l'amorce OPP-HuCλ3' complémentaire de l'extrémité 3' des régions Cλ et apportant le site de restriction BglII et de l'amorce OPP-HuCλ5' complémentaire de l'extrémité 5' des régions Cλ et apportant le site de restriction XhoI.

Les séquences des deux amorces sont les suivantes :
* OPP-HuCλ3': (site BglII souligné)
   identifiée dans la liste des séquences en annexe sous le numéro SEQ ID NO : 6
* OPP-HuCλ5' : (site XhoI souligné)
   identifiée dans la liste des séquences en annexe sous le numéro SEQ ID NO : 7

Après coupure par les enzymes BglII et XhoI, la séquence Cλ amplifiée est insérée entre les sites Xhol et BglII du plasmide pBCκ, préalablement délété du gène Cκ par traitement par les enzymes BglII et XhoI et purification du fragment plasmidique de 7,8 kb.

Le plasmide obtenu est dénommé pBCλ.

L'insertion de la région constante λambda a été vérifiée par séquençage du plasmide pBCλ en présence des deux amorces OPP-HuCλ3' et OPP-HuCλ5'.

La cassette chaîne lambda (Cλ) est destinée au clonage des parties variables de chaînes légères de type lambda.

Ces parties variables sont amplifiées par PCR en utilisant d'une part une amorce (OPP-HuVλ5') qui hybride au niveau de la charpente 1 des chaînes légères et qui permet de reconstituer un site SacI, et d'autre part une amorce (OPP-HuVλ3') qui est quasiment complémentaire de l'amorce OPP-HuCλ5' et qui permet de reconstituer un site Xhol.

Les séquences de ces amorces sont les suivantes :
* OPP-HuVλ5': (site SacI souligné)
   qui est identifiée dans la liste des séquences en annexe sous le numéro SEQ ID NO : 8
* OPP-HuVλ3': (site XhoI souligné)
   qui est identifiée dans la liste des séquences en annexe sous le numéro SEQ ID NO : 9

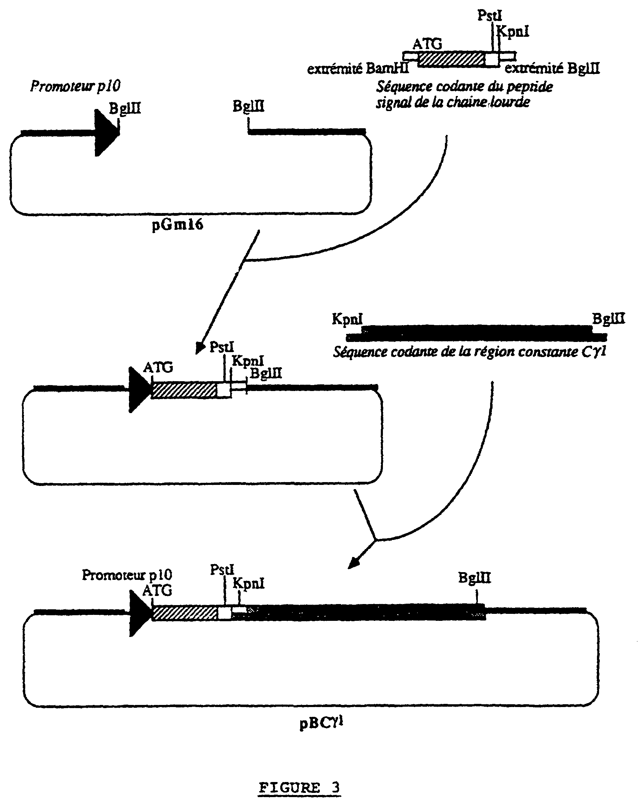

### Exemple 3 - Cassette chaîne lourde γ1 (pBCγ1) (figure 3) :

### a- Plasmide de transfert :

Le plasmide pGml6 [BLANC et al, Virology, 192, 651-654, (1993)] dérive d'un plasmide dans lequel a été cloné le fragment EcoRI-P du baculovirus AcMNPV contenant le gène p10. La quasi-totalité de la séquence codante a été délétée et remplacée par un site BglII permettant l'insertion de séquences à exprimer sous le contrôle du promoteur p10.

### b- Le peptide-signal :

La séquence codante du peptide-signal pour la sécrétion de la chaîne lourde est une séquence artificielle, qui code pour un peptide ayant deux caractéristiques favorisant la sécrétion : hydrophobicité globale (cette caractéristique est commune à tous les peptides-signal), et présence de la séquence Val-His-Ser en amont du site de coupure par la "Signal-Peptidase".

La séquence codante de ce peptide-signal est la suivante :

Elle est identifiée dans la liste des séquences en annexe sous le numéro SEQ ID NO : 10

Elle a été synthétisée chimiquement sous forme de brins complémentaires, de manière à ce qu'elle puisse être insérée dans un site BglII (figure 3). Les conditions d'appariement et de ligation sont identiques à celles utilisées pour le clonage de la séquence codante du peptide-signal utilisée pour la chaîne légère.

### c- Régions constantes humaines :

- IgG1 (Cγ1)
   Le cDNA de la séquence codante de la région Cγ1 humaine a été amplifié par PCR en utilisant les amorces suivantes :
   * HuCγ1BAC : (site KpnI souligné).
      Cette amorce correspond à une séquence consensus des régions JH humaines (extrémités 3' des régions variables des chaînes lourdes humaines), et comprend un site KpnI.
      Elle est identifiée dans la liste des séquences en annexe sous le numéro SEQ ID NO : 11
   * HuCγ1FOR : (site BglII souligné)
      est identifiée dans la liste des séquences en annexe sous le numéro SEQ ID NO : 12

   La séquence a été déterminée à partir de séquences Cγ1 humaines. L'amorce est complémentaire de l'extrémité 3' des Cγ1 humaines, et permet de reconstituer après amplification un site BglII en aval du codon stop.
   La matrice utilisée pour amplifier la région Cγ1 humaine est le même mélange d'ADNc que celui utilisé pour l'amplification des séquences codantes Cκ et Cλ
   Le produit d'amplification a été séquencé et cloné dans le vecteur de transfert pGml6 portant la séquence codant pour le peptide-signal. La construction obtenue a été appelée pBCγ1 (figure 3).
   Pour l'amplification des régions constantes des immunoglobulines IgG2, IgG3, IgG4, IgE, IgM et IgA, on utilise comme amorce 5' l'amorce HuCγ1BAC ci-dessus associée respectivement aux amorces 3' suivantes :
- IgG2 :
   HuCγ1FOR,
- IgG3 :
   HuCγ1FOR,
- IgG4 :
   HuCγ1FOR,
- IgE : identifiée dans la liste des séquences en annexe sous le numéro SEQ ID NO : 13
- IgM : identifiée dans la liste des séquences en annexe sous le numéro SEQ ID NO : 14
- IgA : identifiée dans la liste des séquences en annexe sous le numéro SEQ ID NO : 15.

Pour les régions constantes des IgG1, 2, 3 et 4, la même amorce est utilisée en 3' en raison de la grande conservation de séquences entre les différentes sous-classes dans cette partie. Pour les amorces utilisées pour les IgE, les IgM et les IgA, les sites de coupure BglII introduits sont soulignés.

### Exemple 4 - Expression d'un anticorps chimère (souris-Homme) dans des cellules d'insecte infectées par un vecteur conforme à l'invention :

Le MAb K20 est un anticorps murin produit par un hybridome. Il est dirigé contre la sous-unité β des récepteurs CD29 des lymphocytes [BOUMSELL et al., J. Exp. Med. 152, p. 229 (1980)]. Un baculovirus recombinant conforme à l'invention a été utilisé pour exprimer un anticorps K20 chimère ayant les régions variables du K20 d'origine et les régions constantes humaines provenant des cassettes pBCκ et pBCγ1.
1. Clonage de la région variable de la chaîne légère κ de K20 :
   L'ARN total de l'hybridome a été extrait à l'aide du kit "RNA extraction Kit" de PHARMACIA, et une transcription inverse a été réalisée en utilisant l'amorce VKFOR (First-Strand cDNA Synthesis kit ; PHARMACIA) :
      * VKFOR : (site Xhol souligné)
         identifiée dans la liste des séquences en annexe sous le numéro SEQ ID NO : 16

      Cette amorce est complémentaire de la séquence consensus à l'extrémité 3' de la région variable Vκ des gènes murins. Elle est destinée à amplifier les Vκ réarrangées avec les jonctions Jκ1 ou Jκ2 (qui sont les plus répandues), mais également celles réarrangées avec les jonctions Jκ4 ou Jκ5.
      Le cDNA a été amplifié par PCR en utilisant d'une part l'amorce VκFOR et d'autre part l'amorce Vκ2BAC:
      * Vκ2BAC : (site SacI souligné)
         identifiée dans la liste des séquences en annexe sous le numéro SEQ ID NO : 17.

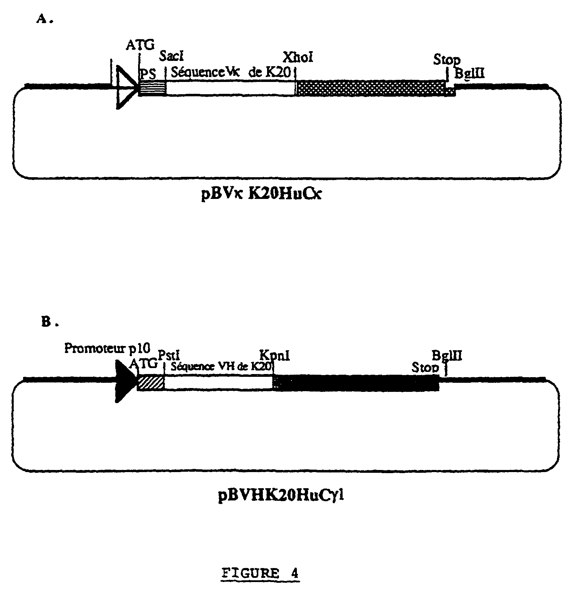

      La séquence de cette amorce est identique à une séquence précédemment publiée [WINTER et CLACKSON, Nature, 352, p 624, (1991)]. VK2BAC est capable d'amplifier les régions variables murines Vκ3, Vκ4 et Vκ6.
      Après amplification de la région VκK20, une digestion SacI-Xhol du produit d'amplification a été réalisée et le fragment obtenu a été cloné entre les sites SacI et Xhol du plasmide chaîne légère pBCκ pour donner le plasmide pBVκK20HuCκ (figure 4A).
2- Clonage de la région variable de la chaîne lourde γ1 de K20 :
   La transcription inverse sur l'ARN total extrait de l'hybridome producteur de K20 a été réalisée en utilisant l'amorce VHFOR. Cette même amorce, et l'amorce VH1BAC ont par la suite servi à amplifier la région VH à partir de l'ADNc :
   * VHFOR : (Site KpnI souligné).
      identifiée dans la liste des séquences en annexe sous le numéro SEQ ID NO : 18
   * VH1BAC : (Site PstI souligné)
      identifiée dans la liste des séquences en annexe sous le numéro SEQ ID NO : 19.

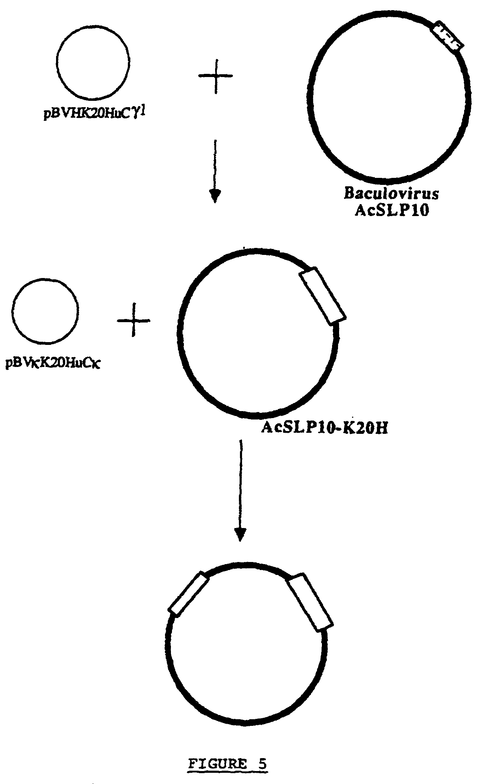

   VHFOR est identique à une amorce décrite par ORLANDI et al. (1989. Proc. Natl. Acad. Sci. USA 86, 3833-3837), la seule différence se situant au niveau du "a" qui remplace un "C" (cf. site KpnI souligné ci-dessus).
   Après amplification et digestion par PstI et KpnI, la région VH de K20 a été insérée dans le plasmide chaîne lourde au niveau des sites PstI-KpnI. Le plasmide chargé a été appelé pBVHK20HUCγ1 (figure 4B).
3- Construction d'un virus recombinant produisant le K20 chimère (figure 5):
   a- Insertion de la chaîne lourde :
      Le plasmide chargé pBVHK20HUCγ1 a été utilisé en cotransfection avec l'ADN d'un baculovirus modifié appelé AcSLp10 [CHAABIHI et al., J. Virol., 67, 2664-2671 (1993)], qui est dépourvu du gène polyédrine (promoteur+séquence codante), mais porte la séquence codante de la polyédrine sous contrôle du promoteur P10, dans le locus naturel P10. Ce virus produisant des polyédres dans les cellules infectées, la recombinaison au niveau du locus P10 peut ainsi être facilement détectée. Les conditions de cotransfection sont les suivantes : 500 ng d'ADN viral sont mélangés avec 5 µg d'ADN plasmidique et 40 µl de solution DOTAP (BOEHRINGER) dans 3 ml de milieu de culture sans sérum pour cellules d'insectes. Ce mélange est utilisé pour recouvrir 4 x 10⁶ cellules Sf9 (ATCC35CRL 1711) ; après 4 heures de contact, le mélange de cotransfection est remplacé par 4 ml de milieu complet et l'incubation est faite à 27°C pendant 5 jours.
      A la suite de la cotransfection, le virus produisant la chaîne lourde de l'anticorps K20 chimère sous le contrôle du promoteur P10 a été purifié par la technique des plages de lyse. Ce virus a été appelé AcSLp10-K20H.
   b- Insertion de la chaîne légère :
      Le plasmide chargé pBVκK20HUCκ a été utilisé en cotransfection avec l'ADN du baculovirus modifié AcSLp10-K20H.
      Les doubles recombinants ont été sélectionnés par la technique de la dilution limite, associée à l'ELISA et/ou à l'hybridation moléculaire.
      Après la cotransfection, on effectue une gamme de dilutions du surnageant infectieux, et chaque dilution est utilisée pour l'infection de cellules d'insectes. Trois jours après l'infection, les surnageants sont testés en ELISA pour rechercher la présence d'anticorps de type humain correctement assemblés. Les surnageants des puits les plus positifs pour les dilutions les plus importantes sont à leur tour dilués et utilisés pour infecter d'autres cultures ; après quelques cycles dilution/infection, les surnageants enrichis en baculovirus produisant l'anticorps entier sont étalés sur un tapis cellulaire, et clonés par la méthode des plages de lyse.

### Exemple 5 - Production et purification de l'anticorps K20 :

Le virus double-recombinant a été amplifié par une série de passages sur des cellules d'insecte en culture. Le stock viral a été ensuite utilisé pour infecter une culture agitée en spinner (500 ml de culture à 10⁶ cellules par ml).

Après 72 heures d'infection, la culture est récoltée et centrifugée à 1000 g pour clarifier le surnageant. Ce dernier a été concentré jusqu'au 1/3 de son volume initial par centrifugation à travers une membrane ayant un seuil de coupure de 30 kDa (CENTRIPEP 30, Amicon) (1ère centrifugation : 1000g, 20°C, 30 minutes ; élimination du filtrat ; 2ème centrifugation : 1000g, 20°C, 20 minutes).

La solution a été équilibrée dans un tampon de fixation sur protéine A, par dilution dans ce tampon suivie d'une nouvelle concentration par centrifugation (tampon de dilution : Glycine 1,5M, NaCl 3M; pH 8,9). La solution équilibrée est ensuite passée dans une colonne de protéine A, elle même équilibrée dans le même tampon que la solution de K20. Après rinçage de la colonne, l'anticorps est élue par un tampon d'élution (Acétate 0,1M, NaCl0,5M; pH3). L'élution est suivie en mesurant la DO à 280 nm. Les fractions contenant l'anticorps ont été mélangées et concentrées par centrifugation à travers une membrane ayant un seuil de coupure de 10 kDa (CENTRIPEP 10, AMICON). La solution concentrée est diluée avec du PBS puis reconcentrée de la même manière. La solution d'anticorps obtenue est conservée à +4°C dans le PBS (137 mM NaCl ; 2,7 mM KCl ; 4,3 mM Na₂HPO₄-7H₂O; 1,4 mM KH₂PO₄.

La qualité de l'anticorps a été contrôlée par électrophorèse sur gel de polyacrylamide-SDS, en utilisant comme témoin une IgG1 humaine du commerce (SIGMA). Cette expérience a montré que l'anticorps chimère non-réduit (ponts disulfure intacts) migre au même niveau que l'anticorps humain témoin.

Après traitement par le dithiotréitol (DTT), on observe l'apparition de deux bandes correspondant aux chaînes lourdes et légères, et migrant au même niveau que les chaînes de l'anticorps humain témoin également réduit au DTT.

Pour confirmer les résultats précédents, les protéines des fractions comprenant l'anticorps ont été transférées sur une membrane de nitrocellulose, et les chaînes H et L ont été détectées par des anticorps spécifiques des région Cγl et Cκ humaines.

Cette expérience démontre que l'anticorps produit par les cellules d'insectes est bien constitué de chaînes H et L, et que les parties constantes de ces chaînes sont reconnues par des anticorps spécifiques.

### Exemple 6 - Test de l'activité et de la spécificité du K20 produit par les cellules d'insectes :

L'anticorps monoclonal K20 est dirigé contre la sous unité β1 des intégrines humaines (récepteur CD29) localisée à la surface des lymphocytes. La fixation de K20 sur la molécule CD29 inhibe la prolifération des lymphocytes T4 humains activés [GROUX et al., Nature, 339, 152-154, (1989)]. L'effet suppressif de K20 sur la prolifération cellulaire T permet d'envisager l'utilisation de cet anticorps dans la prévention des rejets de greffes. Pour tester l'activité de l'anticorps K20 chimérique produit conformément à l'invention, deux types d'expériences ont été réalisés : l'immunofluorescence et l'inhibition de la prolifération lymphocytaire T. Les protocoles expérimentaux suivis ont pour l'essentiel été précédemment décrits [GROUX et al. Nature 339, 152-154 (1989); TICCHIONI et al. Journal of Immunology 151, 119-127 (1993)].

### 1- Expériences d'imunofluorescence :

Des lymphocytes humains portant le récepteur CD29 ont été fixés sur lame de verre puis incubés en présence du R20 chimérique produit conformément à l'Invention, du K20 murin d'origine, ou du tampon seul.

Après une série de rinçages, on rajoute ou bien un anticorps secondaire fluorescent spécifique, du K20 chimérique produit conformément à l'Invention, ou bien un anticorps secondaire fluorescent spécifique du K20 murin d'origine.

Après un nouveau rinçage, les préparations ont été observées au microscope pour visualiser la fluorescence. Ceci a montré que le K20 chimérique produit conformément à l'Invention se fixe de manière spécifique sur les lymphocytes portant le CD29, tout comme le témoin positif K20 murin d'origine ; aucune fluorescence n'a été détectée en l'abscence des anticorps K20.

### 2- Inhibition de la prolifération des lymphocytes CD4⁺ par K20 :

Des lymphocytes humains CD4⁺ ont été activés par un anticorps anti-CD3 en présence d'interleukine-2. Les anticorps K20 chimérique conforme à l'Invention, ou K20 murin d'origine ont été rajoutés (20 µg/ml) pour vérifier leur effet sur la prolifération lymphocytaire. Une expérience avec un anticorps non-inhibiteur [GROUX et al. Nature, 339, p 152. (1989)] a été réalisée pour servir de témoin négatif.

La prolifération est mesurée en comptant la quantité de thymidine ³H incorporée après 4 jours de culture suite aux traitements par les anticorps. On observe 50 à 70% d'inhibition de la prolifération quand les cellules ont été incubées avec l'anticorps K20 produit conformément à l'Invention.

L'anticorps K20 murin d'origine (témoin positif) inhibe également la prolifération des lymphocytes activés dans les mêmes proportions : 50 à 70%.

### Exemple 7 - Comparaison de l'efficacité du promoteur Syn et du promoteur polyédrine :

Deux virus double-recombinants ont été construits selon le protocole décrit dans les exemples 1 à 4 ci-dessus :
- dans l'un d'entre eux (virus 1) la chaîne lourde est sous contrôle du promoteur P10, et la chaîne légère est sous contrôle du promoteur polyédrine ;
- dans l'autre, (virus 2) la chaîne lourde est sous contrôle du promoteur P10, et la chaîne légère est sous contrôle du promoteur Syn.

La quantité d'anticorps sécrétée par des cellules infectées par l'un ou l'autre de ces deux virus est déterminée comme décrit à l'exemple 5.

Les résultats sont illustrés par le Tableau I ci-dessous, qui indique la quantité d'anticorps sécrétés (en µg/ml) à différentes périodes après l'infection (p.i.):

**TABLEAU I**

| Virus | 12 h p.i. | 24 h p.i. | 48h p.i. |
|---|---|---|---|
| 1 | 0,31 | 0,96 | 8,9 |
| 2 | 1,24 | 2,72 | 9,9 |

Ces résultats montrent que la sécrétion d'anticorps est plus précoce et plus importante lorsque le promoteur Syn est utilisé.

## Revendications

1. Baculovirus recombinant, constituant un vecteur d'expression utilisable pour la production d'immunoglobulines dans une cellule d'insecte, et **caractérisé en ce qu'**il comprend :
- une cassette d'expression comprenant une séquence codant pour au moins une partie constante ou variable d'une chaîne H d'une immunoglobuline, laquelle séquence est placée sous contrôle transcriptionnel d'un premier promoteur de baculovirus,
- une cassette d'expression comprenant une séquence codant pour au moins une partie constante ou variable d'une chaîne L d'une immunoglobuline, laquelle séquence est placée sous contrôle transcriptionnel d'un second promoteur de baculovirus;
le premier et le second promoteur étant deux promoteurs différents, et étant situés dans deux loci différents.

2. Baculovirus recombinant selon la revendication 1, **caractérisé en ce que** l'un des promoteurs est situé à l'emplacement occupé chez le baculovirus sauvage, par le promoteur de la polyédrine et l'autre est situé à l'emplacement occupé chez le baculovirus sauvage, par le promoteur de la P10.

3. Baculovirus recombinant selon une quelconque des revendications 1 ou 2, **caractérisé en ce que** les deux promoteurs sont des promoteurs forts.

4. Baculovirus recombinant selon la revendication 3, **caractérisé en ce que** au moins l'un des promoteurs est choisi dans le groupe constitué par :
- le promoteur P10 ;
- le promoteur polyédrine
- un promoteur synthétique, dénommé promoteur Syn, et constitué par un fragment d'ADN double-brin dont la séquence, représentée dans la liste de séquence en annexe sous les numéros SEQ ID NO: 1 et SEQ ID NO: 2, est la suivante :

5. Baculovirus recombinant selon une quelconque des revendications 1 A 4, **caractérisé en ce que** chaque cassette d'expression comprend : (i) un promoteur fort de baculovirus, et sous contrôle dudit promoteur (ii) : une séquence codant pour un peptide-signal ; (iii) une séquence codant pour un domaine variable d'immunoglobuline ; (iv) une séquence codant pour un domaine constant d'une chaîne H ou L d'immunoglobuline.

6. Baculovirus recombinant selon la revendication 5, **caractErisE en ce que** la sEquence codant pour un peptide-signal placEe sous controle du premier promoteur, est diffErente de la sEquence codant pour un peptide-signal placEe sous controle du second promoteur.

7. Baculovirus recombinant selon une quelconque des revendications 5 ou 6, **caractErisE en ce qu'**au moins une des séquences codant pour un peptide-signal code pour un peptide qui possède une séquence His-Val-Ser immédiatement en amont du site de coupure utilisé par la signal-peptidase.

8. Baculovirus recombinant selon l'une quelconque des revendications 5 à 7, **caractérisé en ce que** la séquence codant pour le domaine constant d'immunoglobuline est une séquence d'origine humaine.

9. Cellule d'insecte, infectée par un baculovirus recombinant selon une quelconque des revendications 1 à 8.

10. Procédé de préparation d'une immunoglobuline, **caractérisé en ce que** l'on met en culture des cellules d'insecte, selon la revendication 9 et **en ce que** l'on extrait ladite immunoglobuline à partir du milieu de culture.

11. Procédé de préparation d'un baculovirus recombinant selon une quelconque des revendications 1 à 8, lequel procédé est **caractérisé en ce que** :
- l'on prépare un premier plasmide de transfert comprenant une séquence codant pour au moins une partie constante ou variable de chaîne H d'immunoglobuline, sous contrôle transcriptionnel d'un premier promoteur fort d'un baculovirus ;
- l'on prépare un second plasmide de transfert comprenant la séquence codant pour au moins une partie constante ou variable de chaîne L d'immunoglobuline, sous contrôle transcriptionnel d'un second promoteur fort dudit baculovirus ; le premier et le second promoteur étant deux promoteurs différents ;
- l'on procède à la recombinaison homologue de l'un et de l'autre desdits plasmides avec l'ADN d'un baculovirus ;
- après réplication de l'ADN viral dans les cellules transfectées, l'on procède à la sélection des baculovirus recombinants ayant intégré, dans des loci différents, la séquence codant pour au moins une partie de chaîne H et la séquence codant pour au moins une partie de chaîne L d'immunoglobuline.

12. Procédé selon la revendication 11, **caractérisé en ce que** chaque plasmide de transfert utilisé porte un insert comprenant :
- une cassette d'expression telle que définie dans la revendication 5, et de part et d'autre de cette cassette, des séquences de baculovirus homologues de celles des régions flanquant la portion du génome viral en remplacement de laquelle on souhaite insérer ladite cassette.

13. Procédé selon la revendication 12, **caractérisé en ce que** lesdites séquences de baculovirus sont homologues de celles des régions flanquant le gène de la plO, ou homologues de celles des régions flanquant le gène de la polyédrine.

14. Procédé selon la revendication 13, **caractérisé en ce que** l'ADN de baculovirus avec lequel est effectuée la recombinaison homologue des plasmides de transfert est constitué par l'ADN d'un baculovirus préalablement modifié par insertion de deux sites Bsu36I de part et d'autre de la séquence codant pour la protéine P10 (ces deux sites étant les seuls pour l'enzyme concernée dans le génome dudit baculovirus modifié), et digéré par l'enzyme Bsu36I.

## Patentansprüche

1. Rekombinantes Baculovirus, einen Expressionsvektor ausbildend, der für die Produktion von Immunoglobulinen in einer Insektenzelle nutzbar ist, und **dadurch gekennzeichnet, dass** er aufweist:
- eine Expressionskassette, aufweisend eine für mindestens einen konstanten oder variablen Teil einer H-Kette eines Immunoglobulins kodierende Sequenz, die unter der transkriptionalen Kontrolle eines ersten Baculovirus-Promoters steht,
- eine Expressionskassette, aufweisend eine für mindestens einen konstanten oder variablen Teil einer L-Kette eines Immunoglobulins kodierenden Sequenz, die unter der transkriptionalen Kontrolle eines zweiten Baculovirus-Promoters steht;
wobei der erste und der zweite Promoter zwei verschiedene Promoter sind und sich an zwei verschiedenen Loci befinden.

2. Rekombinantes Baculovirus nach Anspruch 1, **dadurch gekennzeichnet, dass** sich einer der Promoter an der beim wilden Baculovirus vom Polyhedrin-Promoter belegten und der andere an der beim wilden Baculovirus vom P10-Promoter belegten Stelle befindet.

3. Rekombinantes Baculovirus nach einem der Ansprüche 1 oder 2, **dadurch gekennzeichnet, dass** die beiden Promoter starke Promoter sind.

4. Rekombinantes Baculovirus nach Anspruch 3, **dadurch gekennzeichnet, dass** mindestens einer der Promoter aus der Gruppe ausgewählt wird, die gebildet wird von:
- dem Promoter P10,
- dem Polyhedrin-Promoter,
- einem synthetischen Promoter, Promoter Syn genannt, der von einem doppelstrangigen DNA-Fragment gebildet wird, dessen Sequenz, die in der anliegenden Sequenzliste unter den Nummern SEQ ID NO: 1 und SEQ ID NO: 2 dargestellt wird, folgende ist:

5. Rekombinantes Baculovirus nach einem der Ansprüche 1 bis 4, **dadurch gekennzeichnet, dass** jede Expressionskassette umfasst: (i) einen starken Baculovirus-Promoter, und unter Kontrolle des besagten Promoters (ii): eine kodierende Sequenz für ein Signalpeptid; (iii) eine kodierende Sequenz für eine variable Immunoglobulindomäne; (iv) eine kodierende Sequenz für eine konstante Domäne einer H- oder L-Immunoglobulinkette.

6. Rekombinantes Baculovirus nach Anspruch 5, **dadurch gekennzeichnet, dass** sich die für ein Signalpeptid kodierende Sequenz unter Kontrolle des ersten Promoters von der für ein Signalpeptid kodierenden Sequenz unter Kontrolle des zweiten Promoters unterscheidet.

7. Rekombinantes Baculovirus nach einem der Ansprüche 5 oder 6, **dadurch gekennzeichnet, dass** mindestens eine der für ein Signalpeptid kodierenden Sequenzen für ein Peptid kodiert, das eine His-Val-Ser-Sequenz unmittelbar vor der von der Signalpeptidase genutzten Spaltstelle aufweist.

8. Rekombinantes Baculovirus nach einem der Ansprüche 5 bis 7, **dadurch gekennzeichnet, dass** die für die konstante Immunoglobulindomäne kodierende Sequenz eine Sequenz menschlichen Ursprungs ist.

9. Von einem rekombinanten Baculovirus infizierte Insektenzelle nach einem der Ansprüche 1 bis 8.

10. Verfahren zur Herstellung eines Immunoglobulins, **dadurch gekennzeichnet, dass** man Insektenzellen nach Anspruch 9 kultiviert, und dadurch, dass man das besagte Immunoglobulin aus dem Kulturmilieu extrahiert.

11. Verfahren zur Herstellung eines rekombinanten Baculovirus nach einem der Ansprüche 1 bis 8, wobei das Verfahren **dadurch gekennzeichnet ist, dass**:
- man ein erstes Transferplasmid herstellt, das eine für mindestens einen konstanten oder variablen Teil der H-Immunoglobulinkette kodierende Sequenz aufweist, unter transkriptionaler Kontrolle eines ersten starken Baculovirus-Promoters;
- man ein zweites Transferplasmid herstellt, das die für mindestens einen konstanten oder variablen Teil der L-Immunoglobulinkette kodierende Sequenz aufweist, unter transkriptionaler Kontrolle eines zweiten starken Promoters des besagten Baculovirus;
wobei der erste und der zweite Promoter zwei verschiedene Promoteren sind,
- man die homologe Rekombination des einen und des anderen der besagten Plasmide mit der DNA eines Baculovirus durchführt;
- man nach Replikation der viralen DNA in den transfizierten Zellen rekombinante Baculoviren selektiert, die an verschiedenen Loci die für mindestens einen Teil der H-Kette kodierende Sequenz und die für mindestens einen Teil der L-Immunoglobulinkette kodierende Sequenz integriert haben.

12. Verfahren nach Anspruch 11, **dadurch gekennzeichnet, dass** jedes verwendete Transferplasmid ein Insert trägt, aufweisend:
- eine Expressionskassette, so wie in Anspruch 5 definiert, und auf beiden Seiten dieser Kassette Baculovirussequenzen, die denen der Regionen entsprechen, die den Abschnitt des viralen Genoms flankieren als dessen Ersatz man die besagte Kassette einfügen möchte.

13. Verfahren nach Anspruch 12, **dadurch gekennzeichnet, dass** die besagten Baculovirussequenzen denen der Regionen entsprechen, die das Gen der p10 flankieren, oder denen der Regionen entsprechen, die das Polyhedrin-Gen flankieren.

14. Verfahren nach Anspruch 13, **dadurch gekennzeichnet, dass** die DNA des Baculovirus, mit dem die homologe Rekombination der Transferplasmide durchgeführt wird, aus der DNA eines Baculovirus besteht, der zuvor durch Insertion von zwei Bsu36I-Stellen beiderseits der für das Protein P10 kodierenden Sequenz (wobei dieses zwei Stellen die einzigen für das betreffende Enzym im Genom des besagten modifizierten Baculovirus sind) modifiziert und vom Enzym Bsu36I verdaut wurde.

## Claims

1. Recombinant baculovirus, constituting an expression vector for use in the production of immunoglobulins in an insect cell and **characterised in that** it comprises:
- an expression cassette comprising a sequence coding for at least one constant or variable region of an immunoglobulin H chain, which sequence is placed under the transcriptional control of a first baculovirus promoter,
- an expression cassette comprising a sequence coding for at least one constant or variable region of an immunoglobulin L chain, which sequence is placed under the transcriptional control of a second baculovirus promoter;
the first and the second promoters being two different promoters, and being situated at two different loci.

2. Recombinant baculovirus according to claim 1, **characterised in that** one of the promoters is situated at the position occupied in wild-type baculovirus by the polyhedrin promoter and the other is situated at the position occupied in wild-type baculovirus by the p10 promoter.

3. Recombinant baculovirus according to either of claims 1 or 2, **characterised in that** the two promoters are strong promoters.

4. Recombinant baculovirus according to claim 3, **characterised in that** at least one of the promoters is chosen from the group constituted by:
- the p10 promoter;
- the polyhedrin promoter;
- a synthetic promoter, designated the Syn promoter, and constituted by a double-stranded DNA fragment whose sequence, represented in the annexed sequence list under the numbers SEQ ID NO: 1 and SEQ ID NO: 2, is as follows:

5. Recombinant baculovirus according to any of claims 1 to 4, **characterised in that** each expression cassette comprises: (i) a strong baculovirus promoter, and under the control of said promoter (ii): a sequence coding for a signal peptide; (iii) a sequence coding for an immunoglobulin variable region; (iv) a sequence coding for a constant region of an immunoglobulin H or L chain.

6. Recombinant baculovirus according to claim 5 **characterised in that** the sequence coding for a signal peptide placed under the control of the first promoter, is different from the sequence coding for a signal peptide placed under the control of the second promoter.

7. Recombinant baculovirus according to either of claims 5 or 6, **characterised in that** at least one of the sequences coding for a signal peptide codes for a peptide which possesses a His-Val-Ser sequence immediately upstream of the cleavage site used by the signal-peptidase.

8. Recombinant baculovirus according to any of claims 5 to 7, **characterised in that** the sequence coding for the constant immunoglobulin region is a sequence of human origin.

9. Insect cell, infected by the recombinant baculovirus according to any of claims 1 to 8.

10. Method for preparing an immunoglobulin, **characterised in that** insect cells according to claim 9 are cultured, and **in that** said immunoglobulin is extracted from the culture medium.

11. Method for preparing a recombinant baculovirus according to any of claims 1 to 8, which method is **characterised in that**:
- a first transfer plasmid is prepared comprising a sequence coding for at least one constant or variable region of immunoglobulin H chain, under the transcriptional control of a first strong promoter of a baculovirus;
- a second transfer plasmid is prepared comprising the sequence coding for at least one constant or variable region of immunoglobulin L chain, under the transcriptional control of a second strong promoter of said baculovirus;
the first and the second promoters being two different promoters;
- the homologous recombination of both said plasmids with the DNA of a baculovirus is carried out;
- following replication of the viral DNA in the transfected cells, a selection is carried out for the recombination baculoviruses having integrated, in different loci, the coding sequence for at least one part of immunoglobulin H chain and the coding sequence for at least one part of immunoglobulin L chain.

12. Method according to claim 11, **characterised in that** each transfer plasmid used carries an insert comprising:
- an expression cassette such as defined in claim 5, and on either side of this cassette, baculovirus sequences homologous with those of the regions flanking the portion of the viral genome to be replaced by the insertion of said cassette.

13. Method according to claim 12, **characterised in that** said baculovirus sequences are homologous with those of the regions flanking the p10 gene, or homologous with those of the regions flanking the polyhedrin gene.

14. Method according to claim 13, **characterised in that** the baculovirus DNA used to carry out the homologous recombination of the transfer plasmids is constituted by the DNA of a baculovirus previously modified by insertion of two Bsu36I sites on either side of the sequence coding for the p10 protein (these two sites being the only ones for this enzyme in the genome of said modified baculovirus), and digested by the Bsu36I enzyme.


### LISTE DE SEQUENCES

(1) INFORMATIONS GENERALES:
   (i) DEPOSANT:
      (A) NOM: PROTEINE PERFORMANCE (SOCIETE ANONYME)
      (B) RUE: ROUTE D'ALES
      (C) VILLE: SAINT-CHRISTOL-LES-ALES
      (E) PAYS: FRANCE
      (F) CODE POSTAL: 30380

      (A) NOM: CERUTTI MARTINE
      (B) RUE: 2997, ROUTE DE MONTEZE
      (C) VILLE: SAINT CHRISTOL-LES-ALES
      (D) ETAT OU PROVINCE: FRANCE
      (E) PAYS: FRANCE
      (F) CODE POSTAL: 30380

      (A) NOM: CHAABIHI HASSAN
      (B) RUE: 30B, AVENUE JULES GUESDE
      (C) VILLE: ALES
      (D) ETAT OU PROVINCE: FRANCE
      (E) PAYS: FRANCE
      (F) CODE POSTAL: 30100

      (A) NOM: DEVAUCHELLE GERARD
      (B) RUE: 137, CHEMIN DE L'ESPERVETTE
      (C) VILLE: SAINT CHRISTOL-LES-ALES
      (D) ETAT OU PROVINCE: FRANCE
      (E) PAYS: FRANCE
      (F) CODE POSTAL: 30380

      (A) NOM: GAUTHIER LAURENT
      (B) RUE: 186, GRANDE RUE
      (C) VILLE: ALES
      (D) ETAT OU PROVINCE: FRANCE
      (E) PAYS: FRANCE
      (F) CODE POSTAL: 30100

      (A) NOM: KACZOREK MICHEL
      (B) RUE: 81, BOULEVARD DE LA LIRONDE
      (C) VILLE: MONTFERRIER
      (D) ETAT OU PROVINCE: FRANCE
      (E) PAYS: FRANCE
      (F) CODE POSTAL: 34980

      (A) NOM: LEFRANC MARIE-PAULE
      (B) RUE: 4, RUE DU VALLON
      (C) VILLE: CLAPIERS
      (D) ETAT OU PROVINCE: FRANCE
      (E) PAYS: FRANCE
      (F) CODE POSTAL: 34830

      (A) NOM: POUL MARIE-ALIX
      (B) RUE: 10, RUE DE DOCTEUR ROUX
      (C) VILLE: MONTPELLIER
      (D) ETAT OU PROVINCE: FRANCE
      (E) PAYS: FRANCE
      (F) CODE POSTAL: 34000
   (ii) TTTRE DE L' INVENTION: BACULOVIRUS RECOMBINANT ET SON UTILISATION POUR LA PRODUCTION D'ANTICORPS MONOCLONAUX
   (iii) NOMBRE DE SEQUENCES: 19
   (iv) FORME DECHIFFRABLE PAR ORDINATEUR
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 76 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 1:
   ATCAAATAAA TAAGTATTTT AAAGAATTCG TACGTATTTT GTATATTAAT TAAAATACTA 60
   TACTGTAAAT AGATCG 76
(2) INFORMATIONS POUR LA SEQ ID NO: 2:
   (i) CARACTERISTIQUES DE LA SEQUENCE:
      (A) LONGUEUR: 80 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 2:
   TAGTTTATTT ATTCATAAAA TTTCTTAAGC ATGCATAAAA CATATAATTA ATTTTATGAT 60
   ATGACATTTA TCTAGCCTAG 80
(2) INFORMATIONS POUR LA SEQ ID NO: 3:
   (i) CARACTERISTIQUES DE LA SEQUENCE:
      (A) LONGUEUR: 57 paires de bases
      (B) TYPE: nucléotide
      (C) NOMBRE DE BRINS: double
      (D) CONFIGURATION: linéaire
   (ii) TYPE DE MOLECULE: ADN
   (xi) DESCRIPTION DE LA SEQUENCE: SEQ ID NO: 3:
   ATGGGATGGA GCTGTATCAT CCTCTTCTTG GTAGCAACAG CTACAGGTGT CCACTCC 57
(2) INFORMATIONS POUR LA SEQ ID NO: 4:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 4:
   AGCTCGAGAT CAAACGG 17
(2) INFORMATIONS POUR LA SEQ ID NO: 5:
   (i) CARACTERISTIQUES DE LA SEQUENCE:
      (A) LONGUEUR: 28 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 5:
   GAAGATCTAA CACTCTCCGC GGTTGAAG 28
(2) INFORMATIONS POUR LA SEQ ID NO: 6:
   (i) CARACTERISTIQUES DE LA SEQUENCE:
      (A) LONGUEUR: 30 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 6:
   CCTGTCAGAT CTATGAACAT TCTGTAGGGG 30
(2) INFORMATIONS POUR LA SEQ ID NO: 7:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 7:
   CCGCCCTCCC TCGAGCTTCA A 21
(2) INFORMATIONS POUR LA SEQ ID NO: 8:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 8:
   CASTCTGAGC TCACKCAG 18
(2) INFORMATIONS POUR LA SEQ ID ND: 9:
   (i) CARACTERISTIQUES DE LA SEQUENCE:
      (A) LONGUEUR: 27 paires de bases
      (B) TYPE: nucléotide
      (C) NOMBRE DE BRINS: sinple
      (D) CONFIGURATON: linéaire
   (ii) TYPE DE MOLECULE: ADN
   (xi) DESCRIPTION DE LA SEQUENCE: SEQ ID NO: 9:
(2) INFORMATIONS POUR LA SEQ ID NO: 10:
   (i) CARACTERISTIQUES DE LA SEQUENCE:
      (A) LONGUEUR: 57 paires de bases
      (B) TYPE: nucléotide
      (C) NOMBRE DE BRINS: double
      (D) CONFIGURATION: linéaire
   (ii) TYPE DE MOLECULE: ADN
   (xi) DESCRIPTION DE LA SEQUENCE: SEQ ID NO: 10:
   ATGGCTGTCC TGGTGCTGTT CCTCTGCCTG GTTGCATTTC CCAGCTGTGT CCACTCC 57
(2) INFORMATIONS POUR LA SEQ ID NO: 11:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 11:
   CAAGGTACCA CGGTCACCGT CTCC 24
(2) INFORMATIONS POUR LA SEQ ID NO: 12:
   (i) CARACTERISTIQUES DE LA SEQUENCE:
      (A) LONGUEUR: 29 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 12:
   GAAGATCTCA TTTACCCGGA GACAGGGAG 29
(2) INFORMATIONS POUR LA SEQ ID NO: 13:
   (i) CARACTERISTIQUES DE LA SEQUENCE:
      (A) LONGUEUR: 28 paires de bases
      (B) TYPE: nucléotide
      (C) NOMBRE DE BRINS: sinple
      (D) CONFIGURATION: linéaire
   (ii) TYPE DE MOLECULE: ADN
   (xi) DESCRIPTION DE LA SEQUENCE: SEQ ID NO: 13:
   GAAGATCTCA TTTACCGGGA TTTACAGA 28
(2) INFORMATIONS POUR LA SEQ ID NO: 14:
   (i) CARACTERISTIQUES DE LA SEQUENCE:
      (A) LONGUEUR: 31 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 14:
   GAAGATCTCA TTTACCGGTG GACTTGTCGT C 31
(2) INFORMATIONS POUR LA SEQ ID NO: 15:
   (i) CARACTERISTIQUES DE LA SEQUENCE:
      (A) LONGUEUR: 31 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 15:
   GAAGATCTCA GTAGCAGGTG CCGTCCACCT C 31
(2) INFORMATIONS POUR LA SEQ ID NO: 16:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 16:
   CCGTTTGATC TCGAGCTTGG TCCC 24
(2) INFORMATIONS POUR LA SEQ ID NO: 17:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 17:
   GACATTGAGC TCACCCAGTC TCCA 24
(2) INFORMATIONS POUR LA SEQ ID NO: 18:
   (i) CARACTERISTIQUES DE LA SEQUENCE:
      (A) LONGUEUR: 29 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 18:
   TGAGGAGACG GTGACCGTGG TACCTTGGC 29
(2) INFORMATIONS POUR LA SEQ ID NO: 19:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 19:
   AGGTSAAMCT GCAGRAGTCW GG 22